# EUROPÄISCHE PATENTSCHRIFT

(11) **EP 3 313 299 B1**
(45) Veröffentlichungstag und Bekanntmachung des Hinweises auf die Patenterteilung: **07.08.2019**
(21) Anmeldenummer: 16731091.1
(22) Anmeldetag: 16.06.2016
(51) Int. Cl.: A61B 17/16

(54) **CHIRURGIE-HANDGRIFF MIT INNENLIEGENDER UND FEDERVORGESPANNTER VERDREHSICHERUNGSEINHEIT**
SURGICAL HANDPIECE WITH INTEGRATED SPRING-BIASED ROTATION LOCK
POIGNÉE CHIRURGICALE AVEC UNITÉ DE VERROUILLAGE ANTIROTATION INTERNE ET SOLLICITÉE PAR RESSORT

(30) Priorität: 29.06.2015 DE 102015110415
(43) Veröffentlichungstag der Anmeldung: 02.05.2018
(73) Patentinhaber: Aesculap AG, 78532 Tuttlingen (DE)
(72) Erfinder: BÜRK, André, 78056 Villingen-Schwenningen (DE)
(74) Vertreter: Winter, Brandl, Fürniss, Hübner, Röss, Kaiser, Polte - Partnerschaft mbB
(86) Internationale Anmeldenummer: PCT/EP2016/063825
(87) Internationale Veröffentlichungsnummer: WO 2017/001202

(56) Entgegenhaltungen:
- EP-A1- 0 837 749
- DE-B3- 10 311 455
- US-A- 5 989 257
- US-B1- 6 209 886

## Beschreibung

Die Erfindung betrifft einen Chirurgie-Handgriff zum drehfesten Aufnehmen eines Handstückschafts und/oder zum drehmomentübertragenden Aufnehmen eines medizinischen Werkzeugs, mit einer Trägerhülse, an der wenigstens ein zum Kuppeln des Handstückschafts vorgesehenes Kupplungsbauteil, wie wenigstens eine Kugel, eine Tonne oder ein Kegel, so ansetzt, dass das zumindest eine Kupplungsbauteil in eine eingekuppelte und eine ausgekuppelte Stellung im Betriebszustand verbringbar ist, wobei das wenigstens eine Kupplungsbauteil mit einer Aktivierungseinheit in Kontakt bringbar ist, die zwischen der Trägerhülse und einer außen liegenden Betätigungshülse angeordnet ist.

Aus dem Stand der Technik, etwa der EP 1 598 023 B1 ist bereits ein medizinisches Handstück bekannt. Dies umfasst ein Schneidwerkzeug mit einem flexiblen Schaft und einem Bohrer, der am distalen Ende des Schafts zum Schneiden eines betroffenen Gebiets bereitgestellt ist, eine rohrförmige Hülle zum Aufnehmen des Schneidwerkzeugs darin, und einem Handstückkörper zum lösbaren Halten des proximalen Endes des Schneidwerkzeugs und Übertragen von Antriebskraft von einer Antriebsquelle auf das Schneidwerkzeug, wobei die Hülle und das Schneidwerkzeug für einen bestimmten Bereich flexibel gemacht sind, wobei die Hülle angepasst ist, um plastisch deformiert zu werden, während der Schaft des Schneidwerkzeugs elastisch deformiert wird. Als besonders ist herausgestellt, dass das medizinische Handstück eine Mehrzahl von Lagern, die in der Hülle angeordnet sind, aufweist, erste zwischengeordnete Glieder aufweist, die rohrförmig und flexibel sind sowie proximal zu, distal zu und zwischen den Lagern angeordnet sind, und zweite zwischengeordnete Glieder vorhanden sind, die rohrförmig und flexibel sind sowie innerhalb der ersten zwischengeordneten Glieder angeordnet sind, wobei die zweiten zwischengeordneten Glieder den Schaft des Schneidwerkzeugs in sich aufnehmen.

Die US 5,8989,257 A offenbart ein chirurgisches Instrument mit einem medizinischen Handgriff. Über eine Eingangswelle kann über den Handgriff ein Drehmoment auf ein ein- und auskuppelbares Werkzeug übertragen werden. Die Kupplung weist dabei Kugeln auf, welche sich bei einem Verriegelungszustand der Kupplung durch Öffnungen einer Trägerhülse radial nach innen erstrecken und in eine umfängliche Nut des einzukupplenden Werkzeugs eingreifen und damit verriegeln. Eine Betätigungshülse ist dafür zwischen einer verriegelten und entriegelten Position axial verschiebbar und wird in die verriegelte Position vorgespannt. Zwei Madenschrauben, welche in zwei diametral gegenüberliegende und radial nach außen zeigende Durchgangsgewinde in der Betätigungshülse eingeschraubt werden, dienen als Verdrehsicherung.

Chirurgie-Handgriffe weisen also Betätigungselemente auf, die auch hülsenartig ausgebildet sein können. Solche Betätigungshülsen müssen aber gesichert werden, damit keine ungewollte Demontage stattfinden kann. Eine Sicherung erfolgt auch über eine axial verschiebbare Betätigungshülse, wobei außen an die Betätigungshülse angrenzende Teile dann einen Abstand aufweisen können, der ein Einklemmen eines OP-Handschuhs ermöglicht. Dies soll aber vermieden werden. Im Idealfall sollen nach außen keine sichtbaren Veränderungen an der Betätigungshülse vorgenommen werden. Trotzdem soll eine beschädigungsfreie Demontage der Betätigungshülse möglich bleiben.

Die bekannten Lösungen mittels einer außen sichtbaren Schraube oder einer auf die Trägerhülse aufgeschraubten Mutter ist ästhetisch und hygienisch nicht ausreichend. So können OP-Handschuhe eingeklemmt werden und eine Sterilisation nur schwerlich erreicht werden. Selbst alternativ angedachte innenliegende Sprengringe helfen nicht weiter eine einfache Demontage zu erreichen. Häufig werden sogar Demontage-Bohrungen einzubringen sein, die eine zusätzliche Eindringstelle für Verschmutzungen nach sich ziehen, was sich negativ auf die Reinigbarkeit auswirkt. Dabei wäre auch zu beklagen, dass ein Einstich die Stabilität der Welle, auf der sich die Betätigungshülse bewegen soll, einschränkt.

Die Lösung einer außen angebrachten Schraube oder Mutter hat nicht nur erheblichen Einfluss auf das Erscheinungsbild, sondern auch negative Auswirkungen auf die Sicherheit. Falls sich die Schraube oder die Mutter löst, ist die Sicherheit der Betätigungshülse nicht mehr gewährleistet. Der Schraubenkopf bietet ferner eine Angriffsfläche für Verschmutzungen und ist somit schlechter bzw. aufwändiger zu reinigen.

Es ist die Aufgabe der vorliegenden Erfindung, die Nachteile aus dem Stand der Technik zu eliminieren oder zumindest zu mildern. Insbesondere soll das Einklemmen eines OP-Handschuhs verunmöglicht werden und eine sichere aber beschädigungsfreie Demontage erleichtert werden. Ferner soll die Sterilisierbarkeit verbessert werden.

Unteraspekte beziehen sich darauf, dass eine axiale und radiale Sicherung der Betätigungshülse zwischen zwei Endpositionen ermöglicht sein soll, eine Drehmomentübertragung von der Betätigungshülse auf ein Anwendungsteil ermöglicht sein soll, eine Betätigung einer Schließhülse für die Schaftkupplung einfach möglich sein soll, eine Betätigung einer Schubstange für einen Sperrmechanismus störungsfrei möglich sein soll, eine Sicherung gegen eine ungewünschte selbsttätige Demontage der Betätigungshülse während der Anwendung, etwa bei Vibrationen, Stößen und/oder Schlägen, vorgehalten werden soll, und eine solche Verbindungstechnik gewählt werden soll, die ein einfaches und zerstörungsfreies Montieren und Demontieren sicher stellt.

Diese Aufgabe wird bei einem gattungsgemäßen Chirurgie-Handgriff erfindungsgemäß dadurch gelöst, dass eine innerhalb der Betätigungshülse vorhandene Verdrehsicherungseinheit im Betriebszustand so federvorgespannt auf die Aktivierungseinheit einwirkt, dass die Aktivierungseinheit relativ zur Trägerhülse verdrehsicher gehalten ist.

Vorteilhafte Ausführungsformen sind in den Unteransprüchen beansprucht und werden nachfolgend näher erläutert.

So ist es von Vorteil, wenn die Betätigungshülse in einem vom Betriebszustand unterschiedlichen Demontagezustand in eine die Verdrehung der Aktivierungseinheit ermöglichende Position verlagerbar ist. So wird auf der einen Seite immer ein störungsfreier Betrieb möglich, aber bei gewolltem Aufsuchen des Demontage-Zustands, auch eine Demontage, etwa bei einer Überholung / einem Service des Chirurgie-Handgriffes, sichergestellt. Für ein effizientes Nutzen des Chirurgie-Handgriffes im Betriebszustand, ist es von Vorteil, wenn die Aktivierungseinheit eine Anschlaghülse und eine davon separate Schließhülse beinhaltet.

Dabei ist es von Vorteil, wenn die Schließhülse eine solche Innenkontur aufweist, dass bei einer axialen Verlagerung der Schließhülse das Kupplungsbauteil zwischen der eingekuppelten und der ausgekuppelten Stellung zwangsverlagert wird. Ein sicheres Ein- und Auskuppeln des Handstückschafts wird dadurch gewährleistet.

Es ist zweckmäßig, wenn die Anschlaghülse axial- und/oder radialfest an der Betätigungshülse angebracht ist. So kann eine Aktivierungsbewegung von der Betätigungshülse verlustfrei an die Anschlaghülse weitergegeben werden.

Ein vorteilhaftes Ausführungsbeispiel ist auch dadurch gekennzeichnet, dass die Betätigungshülse eine Schulter oder einen Boden aufweist, der zur Mitnahme der Schließhülse nach Zurücklegung eines axialen Totwegs ausgelegt ist. So muss erst eine gewollte axiale Verlagerung der Betätigungshülse bewirkt werden, bevor ein Auskuppeln des Handstückschafts erfolgt. Die Betriebssicherheit des Chirurgie-Handgriffs wird dadurch erhöht.

An dieser Stelle sei auch erwähnt, dass die Erfindung selbstverständlich auch den Chirurgie-Handgriff mit eingekuppeltem Handstückschaft und/oder eingekuppeltem Werkzeug betrifft.

Es ist von Vorteil, wenn die Anschlaghülse axialfest mit der Betätigungshülse verbunden ist und radial in 2, 3 oder mehr Positionen verstellbar ist.

Damit die Verdrehsicherungseinheit selber keine Drehbewegungen durchführt, ist es von Vorteil, wenn sie drehfest, aber axialbeweglich an der Trägerhülse geführt ist. Ein gewolltes Aktivieren und Deaktivieren des Demontage-Zustands bzw. des Ermöglichen des Demontage-Zustands, wird dadurch erleichtert.

Ferner ist es von Vorteil, wenn die Verdrehsicherungseinheit einen, zwei, drei oder mehr Führungsarme aufweist, wobei der Führungsarm oder die Führungsarme in einer Führungsnut bzw. in je einer Führungsnut oder einem Führungskanal bzw. je einem Führungskanal, etwa auf der Außenseite der Trägerhülse möglichst spielfrei gelagert ist/sind. Die Montage wird dadurch erleichtert und die Funktion des Handgriffs verbessert.

Es ist von Vorteil, wenn eine Vielzahl von Kupplungsbauteilen nach Art von Kugeln in eine durch jeweils eine Durchgriffsöffnung der Trägerhülse ragende Einkuppelstellung durch axiales Bewegen der Schließhülse verbringbar sind.

Ein vorteilhaftes Ausführungsbeispiel ist auch dadurch gekennzeichnet, dass die Verdrehsicherungseinheit über eine Verdrehverhinderungsfeder / Verdrehsicherungsfeder, die sich an einem trägerhülsenfesten Abschnitt oder Bauteil abstützt, in Formschluss mit der Aktivierungseinheit gezwungen ist. Die Verdrehsicherungseinheit ist dann immer in einer solchen Stellung, dass eine ungewollte und selbst gewollte Drehbewegung der Aktivierungseinheit, insbesondere der Anschlaghülse, verhindert ist. Erst nach gewollter Verlagerung der Verdrehsicherungseinheit, in Richtung proximal, also von der Aktivierungseinheit, insbesondere der Anschlaghülse, weg, wird eine gewollte Verdrehung der Anschlaghülse zur Demontage ermöglicht.

Es ist zweckmäßig, wenn etwa in Axialrichtung weisende, stirnseitige Verdrehsicherungslaschen der Verdrehsicherungseinheit in vorzugsweise gegengleiche, bspw. in Axialrichtung weisende, stirnseitige Verdrehsicherungsausnehmungen im Betriebszustand eingreifen. Ein guter Zusammenhalt der zwei Einzelkomponenten wird dann erreicht, wobei eine Lösbarkeit auch sichergestellt ist.

Es ist von Vorteil, wenn die Verdrehsicherungseinheit eine Übertragungshülse mit zwei sich gegenüberliegenden Führungsarmen aufweist und einen form- und/oder kraftschlüssig an der Übertragungshülse angebrachten Stützring, an den sich die Verdrehverhinderungsfeder abstützt, umfasst.

Für ein effizientes Zusammenspiel der Einzelkomponenten ist es auch von Vorteil, wenn der Stützring wenigstens eine vorzugsweise radial nach innen ragende Stütznase aufweist, weiter vorzugsweise mehrere Stütznasen, die in eine Tasche der Übertragungshülse formschlüssig eingreift bzw. eingreifen. Sind mehrere Stütznasen vorhanden, sind natürlich auch mehrere Taschen vorhanden, wobei jeweils eine Stütznase in je eine Tasche formschlüssig eingreift. Es hat sich als zweckmäßig herausgestellt, die Stütznasen und Taschen über den Umfang gesehen gleichverteilt auszuformen.

Um ein betriebssicheres Koppeln der Einzelbauteile zu ermöglichen, ist es von Vorteil, wenn die Tasche nach Art eines Bajonettverschlusses ausgebildet ist und vorzugsweise einen Rastbereich zum Aufnehmen der Stütznasen aufweist.

Wenn die Verdrehverhinderungsfeder im Wesentlichen außerhalb der Übertragungshülse angeordnet ist, wird die Montage verbessert. Auch wird der Service schneller, kostengünstiger und effizienter durchführbar.

Es ist von Vorteil, wenn in der Trägerhülse wenigstens ein Anschlagsstift eingesetzt ist, der in einer Führungsnut der Anschlaghülse angeordnet ist. Natürlich ist es von Vorteil, mehrere Anschlagsstifte zu nutzen, die je in einer Führungsnut vorhanden sind. Auch hier hat sich eine über den Umfang gesehen durchgeführte Gleichverteilung als vorteilhaft und kippmomentengegenwirkend herausgestellt.

Es ist von Vorteil, wenn auf der Innenseite der Anschlaghülse eine in Umfangsrichtung ausgerichtete innenseitig der Anschlaghülse vorhandene Freigabenut vorhanden ist.

Dabei ist es von Vorteil, wenn die Freigabenut und/oder eine Auswurfnut positionsmäßig und geometrisch so an den Anschlagsstift und/oder einander angepasst ist/sind, dass der Anschlagsstift bei Drehung mit der Anschlaghülse in die Freigabenut eintaucht und bei nachfolgender Axialverlagerung der Anschlaghülse in eine in die Freigabenut mündende Auswurfnut abbiegt, um die Anschlaghülse freizugeben.

Wenn die Freigabenut ungefähr / exakt mittig zwischen den Endpositionen der Anschlaghülse bei deren Axialverlagerung im Betriebszustand angeordnet ist, so muss ein Operateur erst gewollt die Betätigungshülse verlagern und weiter gewollt die zwischen den Endpositionen zu findende Freigabenut aufsuchen, um dann eine Demontage durchzuführen, die im Betriebszustand aber ungewollt ist. Die Sicherheit im (normalen) Betrieb gegen Demontage wird somit erheblich erhöht.

Es ist auch von Vorteil, wenn die Freigabenut in der Mitte der Führungsnut in diese mündet.

Es hat sich als vorteilhaft herausgestellt, wenn der Stützring im Betriebszustand in Anlage mit der Übertragungshülse ist, und diese wiederum an der Anschlaghülse anliegt. Ein exaktes Betätigen ist dann möglich.

Für einen klapperfreien Betrieb ist es von Vorteil, wenn die Anschlaghülse (nur) kraft- und/oder formschlüssig an der Betätigungshülse befestigt ist oder (nur) stoffschlüssig befestigt ist. Auch ein ungewolltes Verdrehen der Einzelteile zueinander wird dann verhindert.

Es ist von Vorteil, wenn auf der Außenseite der Anschlaghülse Montagelaschen, etwa nach Art von Klemmfedern vorhanden sind und/oder Rastnasen vorhanden sind, die in diese kraft- und/oder formschlüssig aufnehmenden Rastkonturen auf der Innenseite der Betätigungshülse greifen. Im ersten Fall wird ein Ausrichten der Betätigungshülse relativ zu den weiter innenliegenden Teilen möglich und bspw. durch einen Stoffschluss der Anschlaghülse mit der Betätigungshülse dann finalisiert, wohingegen im zweiten Fall auch eine nachträgliche Ausrichtung der Betätigungshülse relativ zum restlichen Handstück durch den Operateur erreicht werden kann. Dies kann insbesondere von Vorteil sein, wenn die Betätigungshülse unsymmetrisch, etwa mit Griffmulden ausgebildet ist.

Ein vorteilhaftes Ausführungsbeispiel ist auch dadurch gekennzeichnet, dass die Verdrehsicherungseinheit einen außenseitig des Handgriffs zugänglich angebrachten Sperrsteg, etwa mit Kerben auf dessen Außenoberfläche, beinhaltet. Die Kerben können dann bspw. genutzt werden, um den Angriff eines Werkzeugs zu erleichtern und während des Services die Demontage zu erleichtern.

Ein vorteilhaftes Ausführungsbeispiel ist auch dadurch gekennzeichnet, dass die Verdrehsicherungseinheit eine, zwei, drei oder mehrere Führungsnut(en) aufweist, die mit Führungselementen, welche mit der Trägerhülse verbunden sind, in Eingriff stehen und/oder die Anschlaghülse über eine oder mehrere in die Führungsnut(en) mündende radiale Freigabenut(en) verfügt, die wiederum in eine oder mehrere Freigabenut(en) mündet / münden.

Mit anderen Worten wird die Betätigungshülse über innenliegende Anschlagsstifte in axialer Richtung und radialer Richtung gesichert. In axialer Richtung ist eine Bewegung der Betätigungshülse zwischen der Verriegelungsposition und der Entriegelungsposition möglich. Die Verriegelung funktioniert selbsttätig über eine Feder. Die Entriegelung funktioniert nur, wenn der mit der Betätigungshülse in Reihe geschaltete Sperrsteg freigegeben ist und ein Anwender die Betätigungshülse entgegen der Federkraft zurück schiebt. In radialer Richtung wird die Betätigungshülse über den gesamten Weg gegen Verdrehung gesichert.

Die Betätigungshülse besitzt in ihrem Inneren eine spezielle Anschlaghülse, welche die Verriegelungskontur für die Stifte beinhaltet. In Kombination mit einer Übertragungshülse, die in die Anschlaghülse eingreift, sind eine einfache Montage und eine beschädigungsfreie Demontage möglich. Die Betätigungshülse kann bei der Montage stufenlos in ihrer radialen Lage zur Anschlaghülse und damit zum gesamten Anwendungsteil verstellt werden. Dies ermöglicht bei einem gewinkelten Anwendungsteil die genaue Ausrichtung der Halteposition. Nach der Einstellung der Position wird die Betätigungshülse demontiert und mit der Anschlaghülse fest verbunden, bspw. mittels Laser-Schweißens. Die Einzelteile des Chirurgie-Handgriffs sind vorzugsweise aus einer Stahllegierung, etwa aus einer Edelstahllegierung gefertigt. In die Anschlaghülse integrierte Federelemente fixieren diese in der Betätigungshülse bis zur endgültigen Fixierung.

In einer besonderen Ausführungsform ist die Anschlaghülse über eine Rastkontur mit der Betätigungshülse verbunden. Es erfolgt keine feste Fixierung. Dies ermöglicht dem Anwender die radiale Verstellung der Betätigungshülse, um weitere Haltepositionen des Anwendungsteils einzustellen.

Besonders erwähnenswert ist, dass die in die Betätigungshülse integrierte Anschlaghülse zwischen den beiden Endpositionen eine Entriegelungsposition besitzt. Durch Freigabe einer Übertragungshülse kann die Betätigungshülse einfach montiert und demontiert werden. Zusätzlich ermöglicht die Anschlaghülse eine stufenlose Einstellung der Betätigungshülse bei der Montage. Bei einer weiteren Ausführung ist eine rastende Verstellung der Betätigungshülse durch den Anwender möglich.

Ein Einklemmen von Handschuhen wird ausgeschlossen, da auf außenliegende Anschläge verzichtet wird. Eine gute Reinigbarkeit der Betätigungshülse, weil diese in ihrer Außenkontur einteilig ausgeführt ist, ist die Folge. Eine uneingeschränkte Gestaltung der Außenkontur im Sinne einer vorteilhaften Design-Gestaltung, wird möglich.

Es wird also eine axial verschiebbare Betätigungshülse vorgestellt, welche über innenliegende Anschläge gesichert ist. Die Betätigungshülse lässt sich einfach montieren, in ihrer radialen Ausrichtung einstellen und bei Bedarf beschädigungsfrei demontieren.

Die Erfindung wird nachfolgend mit Hilfe einer Zeichnung näher erläutert. Es zeigen:
- Fig. 1: eine erste Ausführungsform eines erfindungsgemäßen Chirurgie-Handgriffes mit innenliegendem Anschlag in perspektivischer Darstellung,
- Fig. 2: eine zweite Ausführungsform eines erfindungsgemäßen Chirurgie-Handgriffes, bei der die Betätigungshülse mit Griffmulden versehen ist, in einer zur Fig. 1 vergleichbaren Darstellungsweise,
- Fig. 3: eine Seitenansicht des Ausführungsbeispiels aus Fig. 1,
- Fig. 4: einen Längsschnitt entlang der Linie IV durch das Ausführungsbeispiel aus Fig. 3,
- Fig. 5: eine Detaildarstellung des Bereichs V aus Fig. 4,
- Fig. 6: einen Längsschnitt im Bereich von Anschlagsstiften bei verriegelter Kupplung,
- Fig. 7: einen Längsschnitt im Bereich der Anschlagsstifte bei geöffneter Kupplung,
- Fig. 8: einen Längsschnitt im Bereich von Kupplungsbauteilen, nach Art von Kugeln, bei verriegelter Kupplung des Chirurgie-Handgriffs aus Fig. 6,
- Fig. 9: einen Längsschnitt durch den Chirurgie-Handgriff aus Fig. 7, wobei der Schnitt jedoch nun wiederum im Bereich der Kupplungsbauteile / Kugeln angeordnet ist, bei nun geöffneter Kupplung,
- Fig. 10: das Innenleben des Chirurgie-Handgriffs in perspektivischer Darstellung ohne Betätigungshülse bei verriegelter Kupplung,
- Fig. 11: eine Darstellung des Chirurgie-Handgriffs ohne Betätigungshülse bei geöffneter Kupplung,
- Fig. 12: eine Gesamtansicht des Chirurgie-Handgriffs im Längsschnitt, welcher im Bereich der Anschlagsstifte liegt, bei verriegelter Kupplung und einem eingefahrenen Sperrsteg,
- Fig. 13: eine Gesamtansicht des Chirurgie-Handgriffs im Längsschnitt, wobei die Schnittebene im Bereich der Anschlagsstifte liegt und die Kupplung geöffnet ist, wobei ferner der Sperrsteg ausgefahren ist,
- Fig. 14: eine perspektivische Darstellung der in einer Aktivierungseinheit eingesetzten Anschlaghülse,
- Fig. 15: eine perspektivische Darstellung eines in einer Verdrehsicherungseinheit eingesetzten Stützrings,
- Fig. 16: eine perspektivische Darstellung einer in einer Verdrehsicherungseinheit eingesetzten Übertragungshülse,
- Fig. 17: eine Gesamtansicht des Chirurgie-Handgriffs im Längsschnitt, wobei die Schnittebene im Bereich der Anschlagsstifte liegt, bei eingenommener Demontage-Position,
- Fig. 18: eine Vergrößerung des Bereichs XVIII aus Fig. 17, wobei zur Demontage die Betätigungshülse in die Öffnungsposition der Kupplung bewegt wird, dann mit einem speziellen Haltewerkzeug der Sperrsteg fixiert wird, wobei der Sperrsteg dabei seitliche Kerben diesbezüglich aufweist, wobei durch die Fixierung des Sperrstegs die Übertragungshülse in der hinteren / proximalen Position gehalten wird und dann die Betätigungshülse nach vorne geschoben werden kann, bis sich die radiale Freigabenut auf Höhe der Anschlagsstifte befindet,
- Fig. 19: eine Längsschnittausschnittsdarstellung eines erfindungsgemäßen Chirurgie-Handgriffes, wobei die Schnittebene im Bereich der Anschlagsstifte liegt und eine axiale Freigabenut zur Demontage genutzt ist, wobei die Verdrehsicherung der Übertragungshülse nicht mehr in die Anschlaghülse eingreift, weswegen die Betätigungshülse gegen des Uhrzeigersinns drehbar ist, bis die axiale Freigabenut der Anschlaghülse erreicht ist,

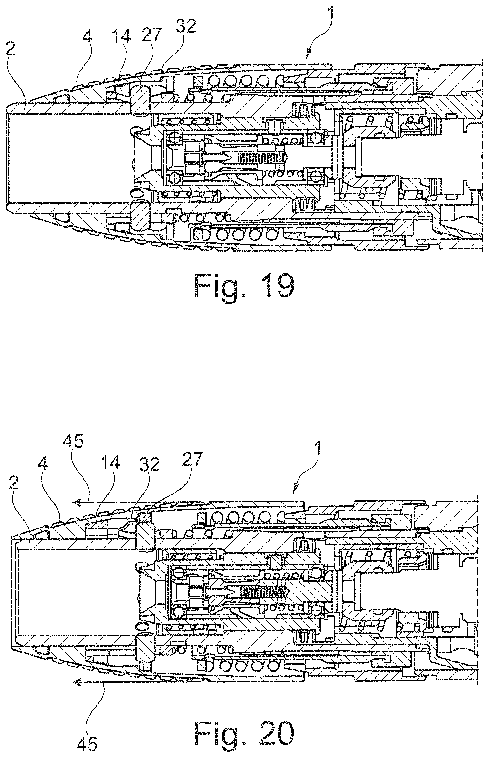

- Fig. 20: eine zu Fig. 19 vergleichbare Darstellung, wobei jedoch über Pfeile eine letztendliche Demontage durch Abziehen angedeutet ist, wobei also in dieser Position die Betätigungshülse nach vorne abgezogen werden kann, danach die Fixierung des Sperrsteges gelöst werden kann und zur erneuten Montage keine Fixierung des Sperrsteges mehr erforderlich ist, wobei ferner die Betätigungshülse in Position der axialen Freigabenut über die Anschlagsstifte geschoben ist, bis die radiale Freigabenut erreicht ist, und desweiteren anschließend die Betätigungshülse im Uhrzeigersinn gedreht wird, bis die Anschlagsstifte an der Wand der Führungsnut anliegen, wobei durch die Federkraft die Übertragungshülse selbsttätig in der Nut für die Verdrehsicherung verriegelt wird,
- Fig. 21: eine zu der in den vorhergehenden Figuren und Ausführungsbeispielen unterschiedliche Ausführungsform, bei der Rastnasen und Rastkonturen an der Anschlaghülse bzw. der Betätigungshülse (wechselweise) ineinander greifen, und
- Fig. 22: eine zu den Fign. 19 und 20 vergleichbare Darstellungsweise bei Nutzens eines gewinkelten Handstücks und einer Betätigungshülse mit Griffmulden (ähnlich zu Fig. 2), wobei der Längsschnitt im Bereich der Anschlagsstifte liegt und die Klemmfedern der Anschlaghülse mit Rastnasen versehen sind, wobei diese in entsprechende Rastkonturen der Betätigungshülse eingreifen, und ferner die Anschlagshülse bei dieser Ausführung mittels eines Sprengrings in axialer Richtung fixiert wird.

Die Figuren sind lediglich schematischer Natur und dienen nur dem Verständnis der Erfindung. Die gleichen Elemente sind mit denselben Bezugszeichen versehen. Merkmale der einzelnen Ausführungsbeispiele können untereinander ausgetauscht werden.

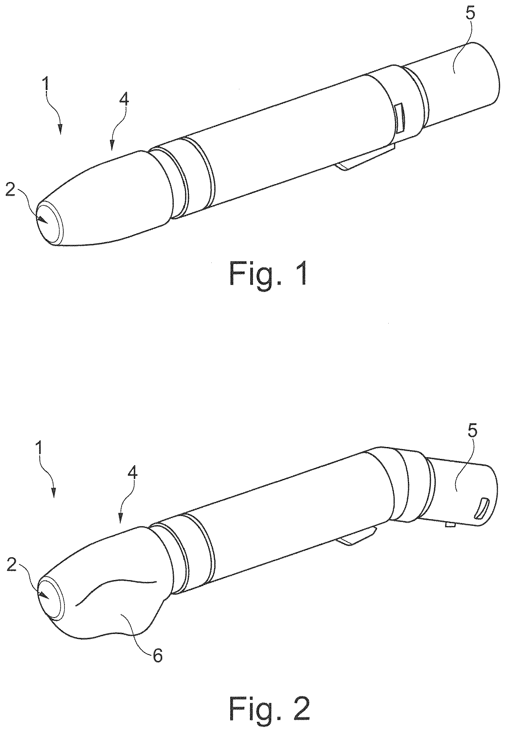

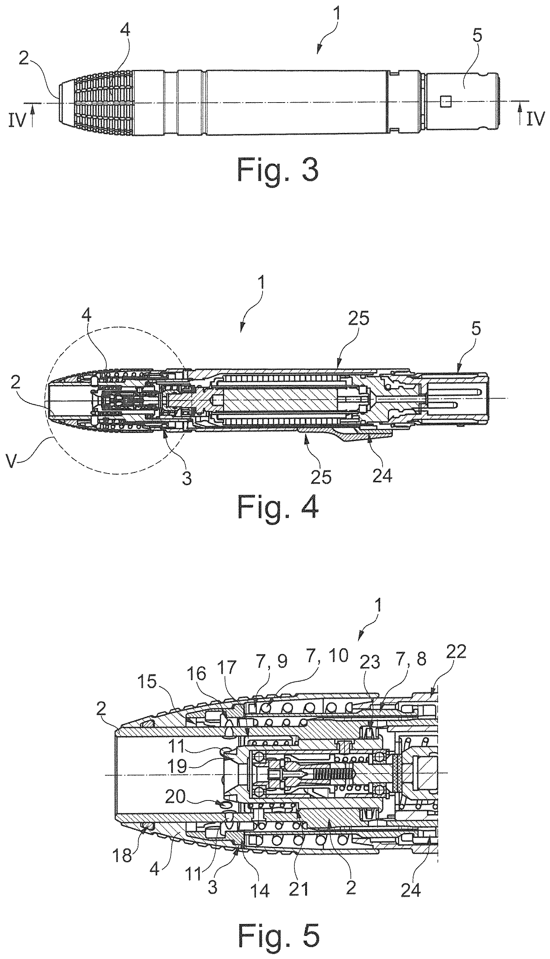

In Fig. 1 ist eine erste Ausführungsform eines erfindungsgemäßen Chirurgie-Handgriffs 1 dargestellt. Der Chirurgie-Handgriff 1 kann auch als Handstück bezeichnet werden. Er ist zum Aufnehmen eines nicht dargestellten Handstückschafts und zum Aufnehmen eines nicht dargestellten medizinischen Werkzeugs, wie eines Fräsers vorgesehen. Im Inneren weist er eine Trägerhülse 2 auf, die ein solches Zentralbauteil ist, relativ zu welchem etwaige bewegbare Elemente bewegbar sind. Die Trägerhülse umgebend ist eine in Fig. 5 gut erkennbare Aktivierungseinheit 3 vorhanden.

Zurückkommend auf Fig. 1, sei erwähnt, dass, obwohl die Aktivierungseinheit 3 als auch die Trägerhülse 2 von einer Betätigungshülse 4 konzentrisch umgeben sind, die Betätigungshülse 4 an einem distalen Ende des Chirurgie-Handgriffs 1 vorhanden ist, an einem proximalen Ende eine Energieversorgungseinheit ankoppelbar ist. Ein entsprechendes Anschlussstück 5 kann, wie in Fig. 2 dargestellt, eine Längsachse aufweisen, die schräg zur Längsachse des restlichen Chirurgie-Handgriffs 1 angestellt ist.

In dem in Fig. 2 dargestellten Ausführungsbeispiel weist die Betätigungshülse 4 wenigstens eine Griffmulde 6 auf. Es sind hier zwei Griffmulden 6 enthalten. Während der in Fig. 1 dargestellte Chirurgie-Handgriff 1 gerade / unabgewinkelt dargestellt ist, ist somit der in Fig. 2 dargestellte Chirurgie-Handgriff 1 gewinkelt ausgebildet. Ein solch gerader Chirurgie-Handgriff 1 ist auch in den Fign. 3 und 4 dargestellt.

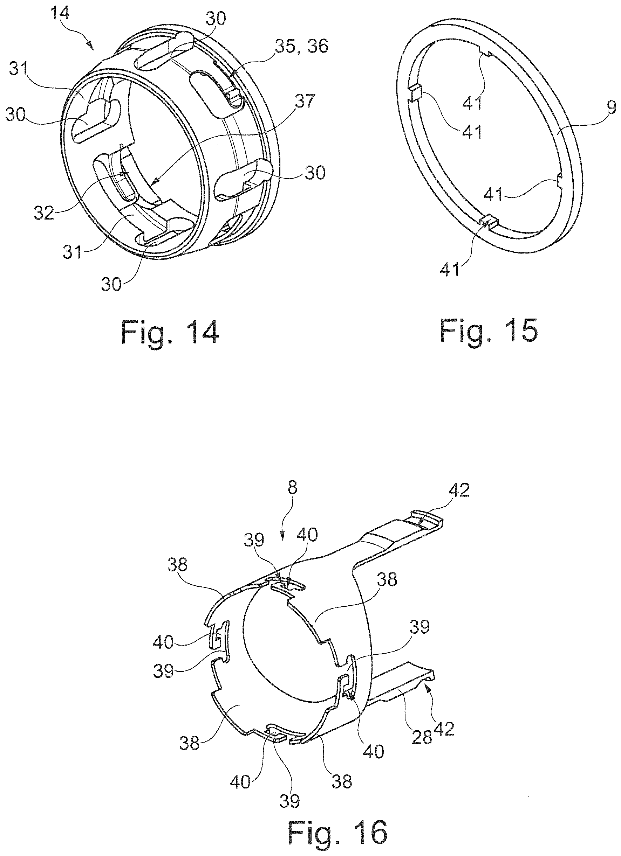

Für das Verständnis der Erfindung besonders gut eignet sich auch die Fig. 5. Dort ist auch eine Verdrehsicherungseinheit 7 dargestellt. Die Verdrehsicherungseinheit 7 umfasst eine Übertragungshülse 8, einen Stützring 9 und eine Verdrehsicherungsfeder / Verdrehverhinderungsfeder 10. Bezüglich des Stützrings 9 und der Übertragungshülse 8 sei auch jetzt schon auf die Fign. 15 und 16 verwiesen.

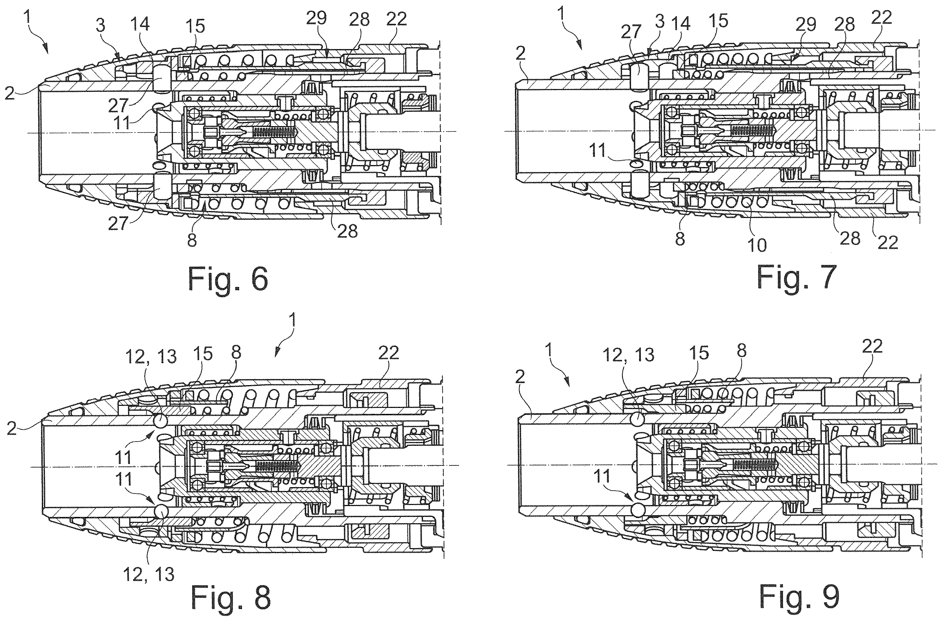

Zurückkommend auf Fig. 5 sei darauf hingewiesen, dass die Betätigungshülse 4 das radial äußerste Bauteil ist. Es sind Durchgriffsöffnungen 11 in einer Wandung der Trägerhülse 2 vorhanden, durch die (in Fig. 5 nicht dargestellte) Kupplungsbauteile 12 (siehe hierfür bspw. Fig. 8 und 9), nach Art von Kugeln 13 (siehe Fign. 6 bis 9) durchgreifen, um einen nicht dargestellten Handstückschaft einzukuppeln und zu halten.

Die Aktivierungseinheit 3 besitzt eine Anschlaghülse 14 und eine davon separate Schließhülse 15. Die Schließhülse 15 ist konzentrisch innerhalb der Anschlaghülse 14 angeordnet und weist eine solch spezielle Innenkontur auf, die in Wirkzusammenhang mit den Kugeln 13 tritt. Proximal der Schließhülse 15 ist eine Stift-Verdrehsicherung 16 vorhanden.

Eine sogenannte Nachfahrhülse 17 ist innerhalb der Trägerhülse 2 vorhanden, um rechtzeitig die Kupplungsbauteile 12 aus den Durchgriffsöffnungen 11 zu verfahren.

In einer umlaufenden Kerbe auf der Innenseite der Betätigungshülse 4 ist ein O-Ring 18 angeordnet.

Im Inneren der Trägerhülse 2 ist auch eine mit dem Bezugszeichen 19 gekennzeichnete Werkzeugkupplung visualisiert. Im Bereich des distalen, stirnseitigen Endes der Werkzeugkupplung 19 sind auch die Durchgriffsöffnungen 11 zum Realisieren einer Kugel-Aufsatzkupplung 20 vorgehalten. Die Trägerhülse 2 kann auch als Träger bezeichnet werden, wobei zwischen der Trägerhülse 2 und der Werkzeugkupplung 19 die Nachfahrhülse 17 angeordnet ist. Zwischen der Nachfahrhülse 17 und der Werkzeugkupplung 19 ist auch eine Druckfeder 21 angeordnet. Am proximalen Ende der Betätigungshülse 4 sitzt auch dichtend eine Abdeckzwischenhülse 22. Diese Abdeckzwischenhülse 22 ist fest mit der Trägerhülse 2 verbunden, insbesondere aufgeschraubt. Eine Spannfeder 23 ist zwischen der Werkzeugkupplung 19 und der Trägerhülse 2 verbaut.

Am proximalen Ende der Übertragungshülse 8 greift eine Sperrhülse 24 an. Diese Sperrhülse 24 kann auch nach Art eines Sperrsteges ausgestaltet sein und bezeichnet werden. Radial außerhalb der Sperrhülse 24 befindet sich ein Betätiger für einen Öffnungsschieber der Werkzeugkupplung 19. Der Betätiger ist mit dem Bezugszeichen 25 versehen.

Zurückkommend auf Fig. 4 sei auch noch ergänzt, dass ein separates Außengehäuse 26 eingesetzt ist, um nach proximal einen Griffbereich zu definieren.

In den Fign. 6 und 7 ist die Aktivierungseinheit 3 mit ihrer Anschlaghülse 14 und ihrer Schließhülse 15 in einer Verriegelungsposition (Fig. 6) bzw. einer Öffnungsposition (Fig. 7) dargestellt. Die beiden Betriebszustände, also die Verriegelungsposition und die Öffnungsposition sind durch einen Anschlag an einem Anschlagstift 27 bestimmt.

In der Verriegelungsposition schlägt ein proximaler Teil der Anschlaghülse 14 an dem Anschlagstift 27 an, wohingegen ein distaler Teil der Anschlaghülse 14 am Anschlagstift 27 in der Öffnungsposition anschlägt. Dadurch bedingt wird auch ein axiales Verlagern der Schließhülse 15 bewirkt, was zu den korrespondierenden Positionen, wie sie in den Fign. 8 und 9 dargestellt sind, führt. Während die Kugeln 13 in der Durchtrittsöffnung 11 durch die Schließhülse 15, nämlich durch deren Innenkontur, in der Darstellung nach Fig. 8 gesperrt ist, also die Kupplung verriegelt ist, sind die Kugeln 13 in der Fig. 9, also in der Öffnungsposition, freigegeben, so dass die Kupplung geöffnet ist.

Die Übertragungshülse 8 weist Führungsarme 28 auf, welche sich in nach außen offenen kanalartigen Führungsnut 29 in Längs- / Axialrichtung erstrecken.

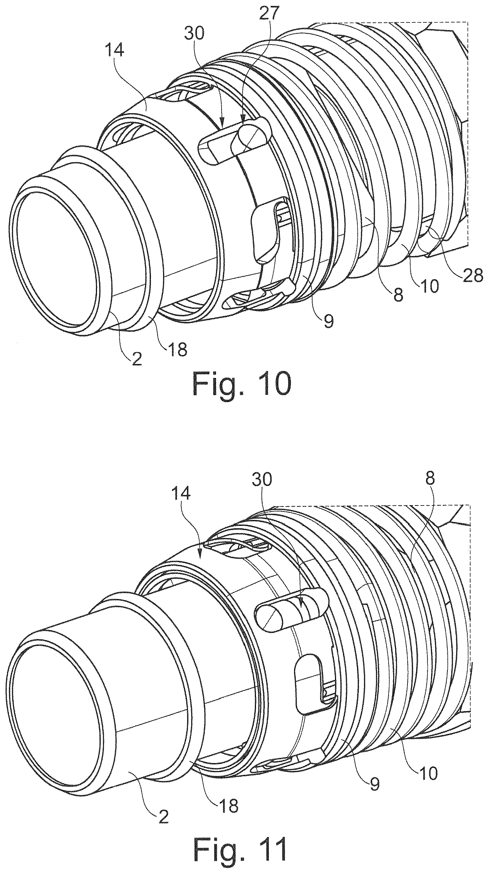

In der Fig. 10 ist die Kupplung verriegelt, wohingegen in Fig. 11 die Kupplung geöffnet ist, derart, dass der Anschlagsstift 27 in einer Anschlagsstiftführungsnut 30 der Anschlaghülse 14 axial beweglich geführt ist, beziehungsweise vice versa. Der Anschlagsstift 27 ist in der verriegelten Position (Fig. 10) im proximalen Endbereich der Anschlagsstiftführungsnut 30 beheimatet, wohingegen er im distalen Endbereich der Anschlagsstiftführungsnut 30 in der kupplungsgeöffneten Position beheimatet ist. Die Verdrehsicherungsfeder 10 ist in drückender Anlage mit dem Stützring 9.

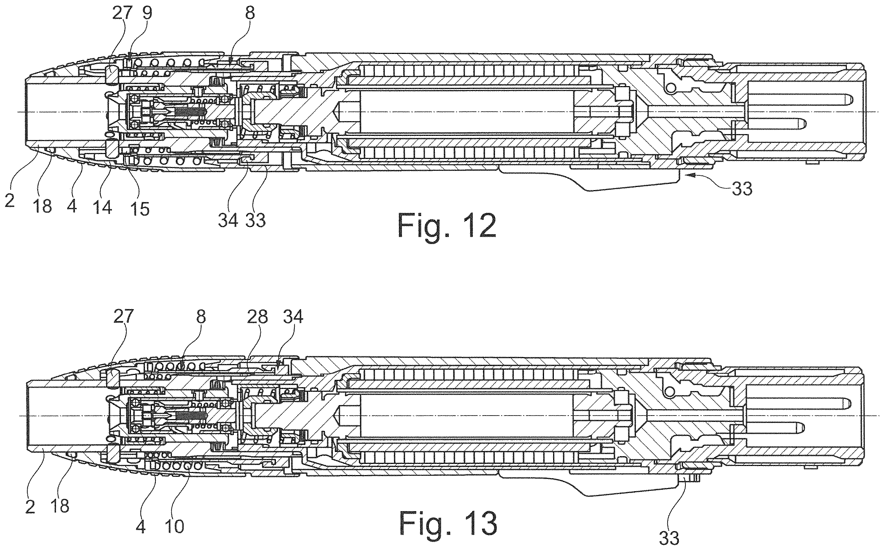

Der Anschlagsstift 27 ist nur so lang, dass er auch in eine in Fig. 14 gezeigte radiale Freigabenut 31 eintauchen kann, die dann in eine orthogonal abzweigende axiale Freigabenut 32 übergeht. Der Anschlagsstift 27 gelangt in die beiden Freigabenuten 31 und 32 nur dann, wenn dies vom Service-Personal gewünscht ist, nämlich dann, wenn der Stützring 9 mit der Übertragungshülse 8 gegen die Verdrehsicherungsfeder 10 nach proximal aktiv, unter Einsatz eines Sperrsteges 33 (siehe Fign. 12 und 13), verfahren wird.

Deshalb wird, wie in Fig. 13 auch gut zu erkennen ist, ein Verbindungselement 34 zum Axialbefestigen eines proximalen Endes eines Führungsarms 28 der Übertragungshülse 8 an einem distalen Ende des Sperrsteges 33 eingesetzt. Zum Anbringen der beiden Bauteile aneinander wird ein Formschluss und/oder ein Kraftschluss genutzt.

Außenseitig der Anschlaghülse 14 ist, wie in Fig. 14 zu erkennen ist, eine MontageHaltelasche 35, nach Art einer Klemmfeder 36 ausgebildet. Diese Klemmfeder 36, insbesondere 2, 3, 4, 5 dieser Klemmfedern 36, sind zum kraftschlüssigen und/oder formschlüssigen in Kontakt gelangen mit der Betätigungshülse 4 bemessen / vorgesehen / abgestimmt.

Am proximalen Ende der Anschlaghülse 14, insbesondere im Bereich des proximalen Ausgangs der axialen Freigabenut 32 sind Verdrehsicherungsnuten 37 vorhanden, die zum Zusammenwirken mit Verdrehsicherungsvorsprüngen der Übertragungshülse 8 vorgesehen sind. Die Verdrehsicherungsnuten 37 und Verdrehsicherungsvorsprünge 38 können auch am jeweils anderen Bauteil angebracht werden. Die Verdrehsicherungsnut 37 kann auch als Verdrehsicherungsausnehmung bezeichnet werden, wohingegen der Verdrehsicherungsvorsprung 38 auch als Verdrehsicherungslasche bezeichnet werden kann.

Es ist noch bedeutsam, dass an der Übertragungshülse 8, insbesondere im distalen Endbereich, für einen bajonettartigen Formschluss vorgehaltene Taschen 39 (siehe Fig. 16) mit Rastbereichen 40 für in diese einbringbaren Stütznasen 41 (siehe Fig. 15) auf der Innenseite des Stützrings 9 vorhanden sind.

Im Bereich des proximalen Endes der Führungsarme 28 (siehe Fig. 16) sind Verbindungselementaufnahmekerben 42 vorhanden.

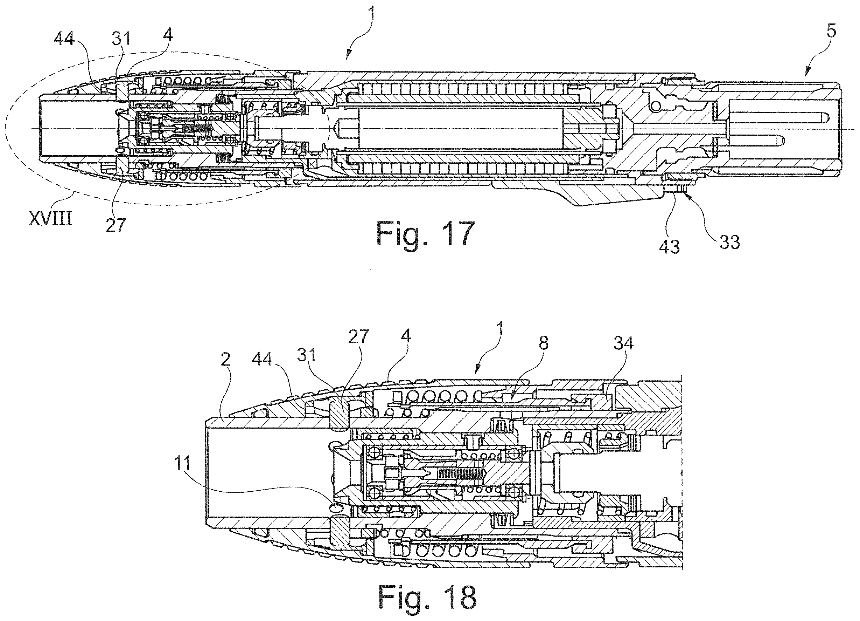

In Fig. 17 sind Werkzeugangriffskerben 43 am Sperrsteg 33 visualisiert und ein Boden der Betätigungshülse 4 mit dem Bezugszeichen 44 referenziert.

In Fig. 18 ist das Eindringen des Anschlagsstiftes 27 in die radiale Freigabenut 31 zu erkennen, genauso wie in Fig. 17, wenn dort auch im etwas kleineren Maßstab. Das weitergehende Bewegen in die axiale Freigabenut 32 und dann das Austreten aus der Anschlaghülse 14 ist in den Fign. 19 und 20 zu erkennen. Die Abzugsrichtung ist mit dem Pfeil 45 visualisiert.

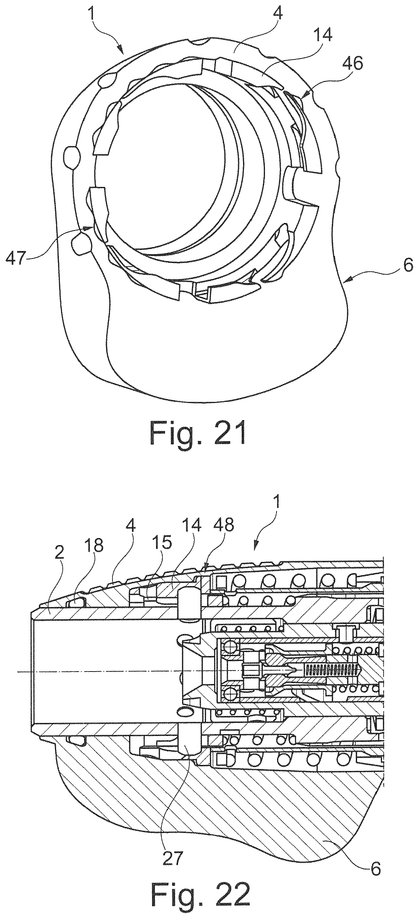

Ein zu den vorher diskutierten Chirurgie-Handgriff-Ausführungsformen unterschiedlicher Chirurgie-Handgriff 1 ist in den Fign. 21 und 22 dargestellt. Eine besondere Abwandlung ist hier das Vorhandensein von Rastnasen 46 an der Anschlaghülse 14, die in vorzugsweise gegengleiche Rastkonturen 47 der Betätigungshülse 4 form- und/oder kraftschlüssig greifen. Die axiale Sicherung der Anschlaghülse 14 wird durch einen Sprengring 48 realisiert.

### Bezugszeichenliste

- 1: Chirurgie-Handgriff
- 2: Trägerhülse
- 3: Aktivierungseinheit
- 4: Betätigungshülse
- 5: Anschlussstück
- 6: Griffmulde
- 7: Verdrehsicherungseinheit
- 8: Übertragungshülse
- 9: Stützring
- 10: Verdrehsicherungsfeder / Verdrehverhinderungsfeder
- 11: Durchgriffsöffnung
- 12: Kupplungsbauteil
- 13: Kugel
- 14: Anschlaghülse
- 15: Schließhülse
- 16: Stift / Verdrehsicherung
- 17: Nachfahrhülse
- 18: O-Ring
- 19: Werkzeugkupplung
- 20: Kugel-Aufsatzkupplung
- 21: Druckfeder
- 22: Abdeckzwischenhülse
- 23: Spannfeder
- 24: Sperrhülse
- 25: Betätiger
- 26: Außengehäuse
- 27: Anschlagsstift
- 28: Führungsarm
- 29: Führungsnut
- 30: Anschlagsstift Führungsnut
- 31: radiale Freigabenut
- 32: axiale Freigabenut
- 33: Sperrsteg
- 34: Verbindungselement
- 35: Montagehaltelasche
- 36: Klemmfeder
- 37: Verdrehsicherungsnut / Verdrehsicherungsausnehmung
- 38: Verdrehsicherungsvorsprung / Verdrehsicherungslasche
- 39: Tasche
- 40: Rastbereich
- 41: Stütznase
- 42: Verbindungselementaufnahmekerbe
- 43: Werkzeugangriffskerbe
- 44: Boden
- 45: Abzugsrichtung
- 46: Rastnase
- 47: Rastkontur
- 48: Sprengring

## Patentansprüche

1. Chirurgie-Handgriff (1) zum drehfesten Aufnehmen eines Handstückschafts und/oder drehmomentübertragenden Aufnehmen eines medizinischen Werkzeugs, mit einer Trägerhülse (2), an der wenigstens ein zum Kuppeln des Handstückschafts vorgesehenes Kupplungsbauteil (12) so ansetzt, dass das zumindest eine Kupplungsbauteil (12) in eine eingekuppelte und eine ausgekuppelte Stellung im Betriebszustand verbringbar ist, in welchem eine Demontage des Chirurgie-Handgriffs (1) ausgeschlossen ist, wobei das wenigstens eine Kupplungsbauteil (12) mit einer Aktivierungseinheit (3) in Kontakt bringbar ist, die zwischen der Trägerhülse (2) und einer außenliegenden Betätigungshülse (4) angeordnet ist, durch deren Betätigung das wenigstens eine Kupplungsbauteil (12) über die Aktivierungseinheit (3) ein- und/oder auskuppelbar ist, **gekennzeichnet durch** eine innerhalb der Betätigungshülse (4) vorhandene Verdrehsicherungseinheit (7), die im Betriebszustand so federvorgespannt auf die Aktivierungseinheit (3) einwirkt, dass die Aktivierungseinheit relativ zur Trägerhülse (2) verdrehsicher gehalten ist.

2. Chirurgie-Handgriff (1) nach Anspruch 1, **dadurch gekennzeichnet, dass** die Betätigungshülse (4) in einem vom Betriebszustand unterschiedlichen DemontageZustand in eine die Verdrehung der Aktivierungseinheit (3) ermöglichende Position verlagerbar ist.

3. Chirurgie-Handgriff (1) nach Anspruch 1 oder 2, **dadurch gekennzeichnet, dass** die Aktivierungseinheit (3) eine Schließhülse (15) und eine davon separate, diese Schließhülse (15) radial umgreifende Anschlaghülse (14) beinhaltet.

4. Chirurgie-Handgriff (1) nach Anspruch 3, **dadurch gekennzeichnet, dass** die Anschlaghülse (14) axial- und radialfest an der Betätigungshülse (4) angebracht ist.

5. Chirurgie-Handgriff (1) nach einem der Ansprüche 3 oder 4, **dadurch gekennzeichnet, dass** die Anschlaghülse (14) axialfest mit der Betätigungshülse (4) verbunden ist und radial in 2, 3 oder mehr Positionen verstellbar ist.

6. Chirurgie-Handgriff (1) nach einem der Ansprüche 3 bis 5, **dadurch gekennzeichnet, dass** die Betätigungshülse (4) eine Schulter oder einen Boden (44) aufweist, der zur Mitnahme der Schließhülse(15) nach Zurücklegung eines axialen Totwegs ausgelegt ist, um das zumindest eine Kupplungsbauteil (12) auszukuppeln.

7. Chirurgie-Handgriff (1) nach einem der Ansprüche 1 bis 6, **dadurch gekennzeichnet, dass** die Verdrehsicherungseinheit (7) drehfest aber axial beweglich an der Trägerhülse (2) geführt ist.

8. Chirurgie-Handgriff (1) nach Anspruch 7, **dadurch gekennzeichnet, dass** die Verdrehsicherungseinheit (7) einen, 2, 3 oder mehr Führungsarme (28) aufweist, der oder die in einer Führungsnut (29) oder einem Führungskanal der Trägerhülse (2) gelagert ist/sind.

9. Chirurgie-Handgriff (1) nach einem der Ansprüche 1 bis 8, **dadurch gekennzeichnet, dass** die Verdrehsicherungseinheit (7) über eine Verdrehsicherungsfeder (10), die sich an einem trägerhülsenfesten Abschnitt oder Bauteil abstützt, in Formschluss mit der Aktivierungseinheit (3) gezwungen ist.

10. Chirurgie-Handgriff (1) nach einem der Ansprüche 1 bis 9, **dadurch gekennzeichnet, dass** in Axialrichtung weisende, stirnseitige Verdrehsicherungslaschen der Verdrehsicherungseinheit (7) im Betriebszustand in Verdrehsicherungsausnehmungen eingreifen, die an der Aktivierungseinheit (3)ausgebildet oder angeordnet sind.

11. Chirurgie-Handgriff (1) nach einem der Ansprüche 1 bis 10, **dadurch gekennzeichnet, dass** die Verdrehsicherungseinheit (7) eine, zwei, drei oder mehrere Führungsnut(en) aufweist, die mit Führungselementen, welche mit der Trägerhülse verbunden sind, in Eingriff stehen.

12. Chirurgie-Handgriff (1) nach zumindest Anspruch 3 und wahlweise einem der Ansprüche 4 bis 11, **dadurch gekennzeichnet, dass** die Anschlaghülse (14) über eine oder mehrere in Führungsnut(en) mündende radiale Freigabenut(en) verfügt, die wiederum in eine oder mehrere Freigabenut(en) mündet / münden.

13. Chirurgie-Handgriff (1) nach Anspruch 9 und 10, **dadurch gekennzeichnet, dass** durch ein axiales Verschieben der Verdrehsicherungseinheit (7) entgegen der Vorspannkraft der Verdrehsicherungsfeder (10) die Anschlaghülse (14) freigegeben und dadurch ein Verdrehen der Betätigungshülse (4) in die Demontagestellung ermöglicht wird.

14. Chirurgie-Handgriff (1) nach Anspruch 5, **dadurch gekennzeichnet, dass** die Anschlaghülse (14) radial in 2, 3 oder mehr Positionen verrastbar ist.

15. Chirurgie-Handgriff (1) nach Anspruch 3 und Anspruch 10, **dadurch gekennzeichnet, dass** die Verdrehsicherungsausnehmungen an der Anschlaghülse (14) ausgebildet oder angeordnet sind.

## Claims

1. A surgical grip (1) for receiving a handpiece shaft for conjoint rotation therewith and/or for receiving a medical tool in such a way as to transmit torque, comprising a carrier sleeve (2) on which at least one coupling component (12) provided for coupling the handpiece shaft is attached such that the at least one coupling component (12) can be brought to a coupled position and to an uncoupled position in the operating state in which disassembly of the surgical grip (1) is excluded, wherein the at least one coupling component (12) can be brought into contact with an activation unit (3) which is arranged between the carrier sleeve (2) and an outer actuation sleeve (4) by actuation of which the at least one coupling component (12) can be coupled and/or uncoupled via the activation unit (3), **characterized by** a rotation securing unit (7) present inside the actuation sleeve (4) which acts on the activation unit (3) in a spring-biased manner in the operating state such that the activation unit (3) is held securely against rotation relative to the carrier sleeve (2).

2. The surgical grip (1) according to claim 1, **characterized in that** the actuation sleeve (4) can be displaced in a disassembly state different from the operating state to a position enabling rotation of the activation unit (3).

3. The surgical grip (1) according to claim 1 or 2, **characterized in that** the activation unit (3) includes a closing sleeve (15) and a stop sleeve (14) separate therefrom and radially encompassing said closing sleeve (15).

4. The surgical grip (1) according to claim 3, **characterized in that** the stop sleeve (14) is attached to the actuation sleeve (4) in an axially and radially fixed manner.

5. The surgical grip (1) according to any one of the claims 3 or 4, **characterized in that** the stop sleeve (14) is connected to the actuation sleeve (4) in an axially fixed manner and is adapted to be radially adjusted into two, three or more positions.

6. The surgical grip (1) according to any one of the claims 3 to 5, **characterized in that** the actuation sleeve (4) includes a shoulder or a bottom (44) designed for catching the closing sleeve (15) after covering an axial dead travel so as to uncouple the at least one coupling component (12).

7. The surgical grip (1) according to any one of the claims 1 to 6, **characterized in that** the rotation securing unit (7) is guided to be rotationally fixed but axially movable at the carrier sleeve (2).

8. The surgical grip (1) according to claim 7, **characterized in that** the rotation securing unit (7) includes one, two, three or more guiding arms (28) being supported in a guiding groove (29) or a guiding channel of the carrier sleeve (2).

9. The surgical grip (1) according to any one of the claims 1 to 8, **characterized in that** the rotation securing unit (7) is forced into form closure with the activation unit (3) via a rotation securing spring (10) which bears against a portion or component fixed to the carrier sleeve.

10. The surgical grip (1) according to any one of the claims 1 to 9, **characterized in that** front-face rotation securing tabs of the rotation securing unit (7) pointing to the axial direction in the operating state engage in rotation securing recesses which are formed or arranged at the activation unit (3).

11. The surgical grip (1) according to any one of the claims 1 to 10, **characterized in that** the rotation securing unit (7) includes one, two, three or more guiding groove(s) which are engaged in guiding elements connected to the carrier sleeve.

12. The surgical grip (1) according to at least claim 3 and optionally according to one of claims 4 to 11, **characterized in that** the stop sleeve (14) has one or more radial release groove(s) opening into guiding groove(s), with the release groove(s) in turn opening into one or more release groove(s).

13. The surgical grip (1) according to claim 9 and 10, **characterized in that** by axially displacing the rotation securing unit (7) against the biasing force of the rotation securing spring (10), the stop sleeve (14) is released and in this way the actuation sleeve (4) is enabled to rotate into the disassembly position.

14. The surgical grip (1) according to claim 5, **characterized in that** the stop sleeve (14) is adapted to be locked at two, three or more positions.

15. The surgical grip (1) according to claim 3 and claim 10, **characterized in that** the rotation securing recesses are formed or arranged at the stop sleeve (14).

## Revendications

1. Poignée chirurgicale (1) destinée à loger de manière solidaire en rotation une tige porte-instruments et/ou à loger de manière à transmettre un couple de rotation un instrument médical, avec un manchon porteur (2) au niveau duquel au moins une pièce de couplage (12) prévue pour le couplage avec la tige porte-instruments est placée de telle sorte que l'au moins une pièce de couplage (12) peut être amenée dans une position couplée et dans une position découplée dans l'état de fonctionnement dans lequel un démontage de la poignée chirurgicale (1) est exclu, l'au moins une pièce de couplage (12) pouvant être amenée en contact avec une unité d'activation (3) qui est agencée entre le manchon porteur (2) et un manchon d'actionnement (4) qui est situé à l'extérieur et par l'actionnement duquel l'au moins une pièce de couplage (12) peut être couplée et/ou découplée par l'intermédiaire de l'unité d'activation (3), **caractérisée par** une unité de verrouillage antirotation (7) qui est présente à l'intérieur du manchon d'actionnement (4) et qui, dans l'état de fonctionnement, agit de telle manière avec une précontrainte élastique sur l'unité d'activation (3) que l'unité d'activation est maintenue verrouillée en rotation par rapport au manchon porteur (2).

2. Poignée chirurgicale (1) selon la revendication 1, **caractérisée en ce que,** dans un état de démontage différent de l'état de fonctionnement, le manchon d'actionnement (4) peut être déplacé dans une position permettant la rotation de l'unité d'activation (3).

3. Poignée chirurgicale (1) selon la revendication 1 ou 2, **caractérisée en ce que** l'unité d'activation (3) contient un manchon de fermeture (15) et un manchon de butée (14) séparé de celui-ci et entourant de façon radiale ce manchon de fermeture (15).

4. Poignée chirurgicale (1) selon la revendication 3, **caractérisée en ce que** le manchon de butée (14) est monté, solidaire de façon axiale et de façon radiale, sur le manchon d'actionnement (4).

5. Poignée chirurgicale (1) selon l'une quelconque des revendications 3 ou 4, **caractérisée en ce que** le manchon de butée (14) est relié, solidaire de façon axiale, au manchon d'actionnement (4) et est déplaçable de façon radiale dans 2, 3 ou plus positions.

6. Poignée chirurgicale (1) selon l'une quelconque des revendications 3 à 5, **caractérisée en ce que** le manchon d'actionnement (4) comporte un épaulement ou un fond (44) qui est conçu pour entraîner le manchon de fermeture (15) après un recul équivalent à une course morte axiale afin de découpler l'au moins une pièce de couplage (12).

7. Poignée chirurgicale (1) selon l'une quelconque des revendications 1 à 6, **caractérisé en ce que** l'unité de verrouillage antirotation (7) est guidée de manière solidaire en rotation mais mobile de façon axiale au niveau du manchon porteur (2).

8. Poignée chirurgicale (1) selon la revendication 7, **caractérisée en ce que** l'unité de verrouillage antirotation (7) comporte un, 2, 3 ou plus bras de guidage (28) qui sont logés dans une rainure de guidage (29) ou dans un canal de guidage du manchon porteur (2).

9. Poignée chirurgicale (1) selon l'une quelconque des revendications 1 à 8, **caractérisée en ce que** l'unité de verrouillage antirotation (7) est forcée dans un assemblage par concordance de forme avec l'unité d'activation (3) par l'intermédiaire d'un ressort de verrouillage antirotation (10) qui s'appuie à un élément ou tronçon solidaire du manchon porteur.

10. Poignée chirurgicale (1) selon l'une quelconque des revendications 1 à 9, **caractérisée en ce que,** dans l'état de fonctionnement, des attaches de verrouillage antirotation, pointant en direction axiale et situées frontalement, de l'unité de verrouillage antirotation (7) pénètrent dans des évidements de verrouillage antirotation qui sont réalisés ou agencés au niveau de l'unité d'activation (3).

11. Poignée chirurgicale (1) selon l'une quelconque des revendications 1 à 10, **caractérisée en ce que** l'unité de verrouillage antirotation (7) comporte une, deux, trois ou plus rainures de guidage qui sont en prise avec des éléments de guidage qui sont assemblés au manchon porteur.

12. Poignée chirurgicale (1) selon au moins la revendication 3 et au choix l'une quelconque des revendications 4 à 11, **caractérisée en ce que** le manchon de butée (14) dispose d'une ou plusieurs rainures de dégagement radiales, débouchant dans des rainures de guidage, qui débouchent elles-mêmes dans une ou plusieurs rainures de dégagement.

13. Poignée chirurgicale (1) selon la revendication 9 et 10, **caractérisée en ce que,** par une translation axiale de l'unité de verrouillage antirotation (7) contre la force de précontrainte du ressort de verrouillage antirotation (10), le manchon de butée (14) est dégagé et une rotation du manchon d'actionnement (4) dans la position de démontage est ainsi permise.

14. Poignée chirurgicale (1) selon la revendication 5, **caractérisée en ce que** le manchon de butée (14) peut être enclenché de façon radiale dans 2, 3 ou plus positions.

15. Poignée chirurgicale (1) selon la revendication 3 et la revendication 10, **caractérisée en ce que** les évidements de verrouillage antirotation sont réalisés ou agencés au niveau du manchon de butée (14).
